# EUROPÄISCHE PATENTSCHRIFT

(11) **EP 1 537 384 B1**
(45) Veröffentlichungstag und Bekanntmachung des Hinweises auf die Patenterteilung: **14.10.2015**
(21) Anmeldenummer: 03775141.9
(22) Anmeldetag: 26.08.2003
(51) Int. Cl.: G01D 3/08, G01D 18/00, G01N 27/416, G01D 1/00, G01N 27/417, G01N 33/00

(54) **Verfahren zur Funktionsüberwachung von Sensoren**
Method of monitoring sensor function
Procédé de surveillance du fonctionnement de capteurs

(30) Priorität: 29.08.2002 DE 10239610
(43) Veröffentlichungstag der Anmeldung: 08.06.2005
(60) Teilanmeldung: 15183147.6
(73) Patentinhaber: Endress + Hauser Conducta GmbH + Co. KG, 70839 Gerlingen (DE)
(72) Erfinder: WITTMER, Detlev, 75433 Maulbronn (DE); STEINMÜLLER, Dirk, 76139 Karlsruhe (DE); FIKUS, Axel, 04746 Hartha (DE); HAMMELEHLE, Wilfried, 70825 Korntal-Münchingen (DE)
(74) Vertreter: Andres, Angelika Maria
(86) Internationale Anmeldenummer: PCT/EP2003/009438
(87) Internationale Veröffentlichungsnummer: WO 2004/025223

(56) Entgegenhaltungen:
- WO-A-02/054056
- DE-A- 4 221 848
- US-A- 4 532 013
- US-A- 4 638 658
- US-A- 5 273 640
- US-A- 6 076 389
- PATENT ABSTRACTS OF JAPAN vol. 017, no. 643 (P-1651), 29. November 1993 (1993-11-29) -& JP 05 209858 A (NGK INSULATORS LTD), 20. August 1993 (1993-08-20)

## Beschreibung

Die Erfindung betrifft ein Verfahren zur Funktionsüberwachung von Sensoren zur Messung und Überwachung von Zustandsparametern von Flüssigkeiten oder Gase, insbesondere im Bereich der Prozessmesstechnik. Es kann sich hierbei um an sich beliebige elektrochemische, elektrophysikalische oder optische Sensoren, insbesondere potentiometrische oder amperometrische, photometrisch oder spektrometrische Sensoren handeln. Bei den zu messenden und zu überwachenden Zustandsparametern kann es sich beispielsweise um den pH-Wert, den CO₂-, O₂-Gehalt, die Konzentration eines Stoffes in einer wässrigen Lösung oder die elektrische Leitfähigkeit handeln.

Sensoren im Bereich der Prozessmesstechnik unterliegen einer zeitlichen aber auch stark anwendungsbezogenen Alterung. Insbesondere sind Sensoren, welche in flüssigen Medien, zum Beispiel bei der Überwachung von chemischen Prozessen eingesetzt werden, besonderen Beanspruchungen ausgesetzt, so dass die Anforderungen an ihre chemische Beständigkeit und an ihre Temperaturbeständigkeit hoch sind. Ebenso können sich Verschmutzung und Belagsbildung auf dem Sensor in Folge des Medienkontakts störend auswirken und die Standzeit des Sensors verringern. Die Funktionsfähigkeit eines Sensors und seine Standzeit werden durch äußere Einflüsse oder innere, im Sensor vorliegende Einflüsse beeinträchtigt beziehungsweise beeinflusst. Es ist daher nicht möglich, die Standzeit eines Sensors im allgemeinen oder für jeden Sensor im speziellen Anwendungsfall anzugeben, und vorherzusagen, was als nachteilig anzusehen ist.

Bei elektrochemischen, elektrophysikalischen oder optischen Sensoren im Bereich der Prozessmesstechnik werden üblicherweise von Zeit zu Zeit Kalibrierungen durchgeführt, bei denen Mittels entsprechender Normalien eine Zwei- oder Mehrpunktkalibrierung zu Grunde gelegt wird. Die auf diese Weise erhaltenen Kalibrierdaten werden zur Verfügbarkeit für die Messwertauswertung in einem Speicher eines Messwertumformers oder aber des Sensors selbst (siehe DE 102 18 606) abgelegt. Bei der Messwerterfassung und - Auswertung werden dann diese Kalibrierdaten und gegebenenfalls weitere Messdaten, wie zum Beispiel die Temperatur des Messmediums, herangezogen.

Beispielsweise wird eine pH-Messkette, die durch eine pH-Glaselektrode und eine Bezugselektrode gebildet ist, durch die Parameter Nullpunkt und Steilheit der Kurve der Messkettenspannung charakterisiert. Als Glaselektrodenfehler sind Steilheitsfehler, Alkalifehler und Nullpunkt bekannt. Die Lebensdauer einer pH-Elektrode ist daher sowohl von den Einsatzbedingungen, aber auch von der Beständigkeit des verwandten Elektrodenglases abhängig. In der Regel sind Einflüsse auf die sogenannte Membranfläche, also Belagbildung aus Kalk, Gips, Fetten, Eiweiß und dergleichen durch chemische Reinigung behebbar. Nicht behebbar sind jedoch Alterungsprozesse im Inneren des Sensors, die auch bei Lagerung stattfinden. Vor allem aber verringern der Betrieb bei extremen pH-Werten aber bei hohen Temperaturen die Lebensdauer einer Glaselektrode drastisch.

Bezugselektroden hingegen besitzen ein Diaphragma, welches den elektrolytischen Kontakt zwischen einem Bezugselektrolyten und der Messlösung ermöglicht. Fehler können daher zum Beispiel durch Verschmutzung und Blockierung des Diaphragmas oder durch sog. Elektrodenvergiftung aufgrund eindringender Fremdionen in den Bezugselektrolyten hervorgerufen werden. Die Folge ist eine Veränderung der Zellenspannung der Bezugselektrode (der Halbzellenspannung), des sogenannten Bezugspotentials, was sich wiederum als Nullpunktsveränderung der gesamten Messkettenspannung bemerkbar macht.

Es sind daher Verfahren zur Funktionsüberwachung von elektrochemischen Sensoren bekannt, wobei zum Beispiel der Innenwiderstand einer Messelektrode und einer Bezugselektrode in zeitlichen Abständen gemessen wird und bei Überschreiten oder Unterschreiten praxisbezogen definierter Werte eine Alarmmeldung ausgegeben wird. Nach diesen Verfahren wird abgewartet, bis der Sensor einen kritischen Bereich erreicht und dann ausgewechselt werden muss.

Es gibt eine Vielzahl von Verfahren zur Funktionsüberwachung von Sensoren im Bereich der Prozessmesstechnik, wobei stets anhand von Prüfparametern die momentane Funktionserfüllung des Sensors geprüft wird.

Beispielhaft sei DE 42 12 792 C2 und DE 34 19 034 A1 US 4,532,013, JP 520 9858, US 6,076,389, WO 02/054056 und US 5,273,640 genannt.

Der vorliegenden Erfindung liegt die Aufgabe zu Grunde, ein Verfahren zur Funktionsüberwachung mit den Merkmalen des Oberbegriffs des Patentanspruchs 1 dahingehend zu verbessern, dass ausgeschlossen werden kann, dass der Sensor während der folgenden Betriebsperiode bis zur nächsten Funktionsprüfung seine Funktionsfähigkeit verliert, und zwar ohne dass der Sensor lange vor Erreichen seiner Abnutzungsgrenze ausgetauscht wird, so dass die wertvolle Standzeit des Sensors möglichst weitgehend ausgenutzt werden kann.

Diese Aufgabe wird bei einem gattungsgemäßen Verfahren erfindungsgemäß dadurch gelöst, dass dass die erfassten Prüfparameter gespeichert werden und dass zur Durchführung der Funktionsüberwachung eine seitherige zeitliche Entwicklung der gespeicherten Prüfparameter ausgewertet wird und dass daraus die zukünftige zu erwartende Entwicklung des Sensorverhaltens vorhergesagt wird und Informationen über die Dauer des verbleibenden störungsfreien Betriebs des Sensors gewonnen werden.

Der vorliegenden Erfindung liegt die Erkenntnis zugrunde, dass aus der seitherigen zeitlichen Entwicklung von Prüfparametem, die entweder im Rahmen der Messwerterfassung oder während periodischer Prüfzustände, insbesondere während der Kalibriervorgänge, gewonnen werden, in die Zukunft gerichtete Voraussagen über die zu erwartende Entwicklung des Sensorverhaltens gemacht werden können. Es soll also der noch verbleibende "Abnutzungsvorrat" des Sensors ermittelt werden. Anhand dessen können Steuervorgänge ausgelöst oder Entscheidungen über Steuervorgänge getroffen werden, falls dies erwünscht ist.

Dabei können in vorteilhafter Weise erfahrungsgemäß gewonnene Kenntnisse über das Verhalten eines bestimmten Sensortyps bzw. eines bestimmten Prüfparameters bei einem spezifischen Sensortyp verwendet werden, etwa dadurch, dass ein bestimmtes erfahrungsgemäßes Verhalten sehr gut wiedergebende mathematische Funktionen verwendet und deren Parameter ermittelt werden.

Es wäre zumindest grundsätzlich denkbar, dass eine erfahrungsgemäße zeitliche Entwicklung eines Prüfparameters in Form einer Kurve oder in sonstiger Weise für die Funktionsüberwachung zugänglich hinterlegt werden könnte, so dass durch Vergleich der ermittelten und gespeicherten Prüfparameter des zu überwachenden Sensors mit den hinterlegten Daten auf die zu erwartende Entwicklung des Sensorverhaltens geschlossen werden kann. Es könnte also insbesondere eine Aussage gewonnen werden, wie lange der Sensor noch außerhalb eines kritischen Verhaltens, also in einem sicheren Abstand zu einer Abnutzungsgrenze bleiben wird. - Es erweist sich aber demgegenüber als vorteilhaft, wenn zum Auswerten der seitherigen zeitlichen Entwicklung der gespeicherten Prüfparameter nichtlineare Interpolationsverfahren angewendet werden, um Funktionsparameter einer das Sensorverhalten beschreibenden Funktion zu gewinnen. Es wird also durch Interpolation im weitesten Sinne eine Funktion für die Prüfparameter errechnet, deren Werte dann auch für die Zukunft berechnet werden können. Es findet also im weitesten Sinne eine Extrapolation der in der Vergangenheit ermittelten Prüfparameter für die Zukunft statt.

Wie bereits vorstehend angedeutet, erweist es sich als besonders vorteilhaft, wenn für einen bestimmten Sensortyp und gegebenenfalls für einen bestimmten Anwendungsbereich eines bestimmten Sensortyps, etwa im alkalischen Medium, eine auf Erfahrung beruhende Funktion vorgegeben und verwendet wird, deren Funktionsparameter dann aber anhand einer Anzahl von konkreten erfassten Prüfparametem ermittelt werden. Vorzugsweise finden Polynomfunktionen hierfür Verwendung.

Wenn vorstehend von Informationen über die Dauer des verbleibenden störungsfreien Betriebs des Sensors gesprochen wird, so ist dies im weitesten Sinne zu verstehen. Insbesondere könnte nach einer jeweiligen Erfassung eines Prüfparameters oder in zeitlichen Abständen ein erster prädiktiver Wert für eine Abnutzungsgrenze ermittelt werden. An der Abnutzungsgrenze ist der Sensor nach wie vor funktionsfähig, d.h. er befindet sich in einem Betriebszustand, der (noch) durch elektrische Kompensationsmaßnahmen die Funktionalität der Messeinrichtung in gegebenenfalls engeren Grenzen ermöglicht. Jenseits der Abnutzungsgrenze ist dies aber nicht mehr der Fall. Es wird also nach diesem weitergehenden Erfindungsgedanken derjenige Zeitpunkt rechnerisch anhand der erfassten Prüfparameter, insbesondere anhand der daraus gewonnenen Funktion oder im weitesten Sinne durch Extrapolation ermittelt, zu dem der Sensor die vorstehend erläuterte Abnutzungsgrenze erreicht haben wird.

Nach einer weiteren Ausführungsform der Erfindung könnte aber auch geprüft werden, ob vor dem nächsten Erfassen von Prüfparametern, also insbesondere vor dem nächstfolgenden Kalibrierzyklus, die Abnutzungsgrenze erreicht sein wird. Je nach dem kann eine entsprechende Warnmeldung ausgegeben werden, oder es können Maßnahmen verschiedenster Art, beispielsweise automatisierte Reinigungsmaßnahmen, eingeleitet werden.

Nach einer weiteren vorteilhaften Verfahrensvariante kann geprüft werden, ob ein prädiktiv gewonnener Wert des Prüfparamters innerhalb eines Warnbereichs diesseits der bisher definierten Abnutzungsgrenze liegt. Auch in diesem Fall könnten geeignete Informationen angezeigt oder Maßnahmen ausgelöst werden. Die bisher definierte Abnutzungsgrenze kann insbesondere anhand des prädiktiv gewonnenen Werts korrigiert werden.

In besonders bevorzugter Weiterbildung des Erfindungsgedankens wird vorgeschlagen, dass anhand der erfindungsgemäß gewonnenen Informationen über die Dauer des verbleibenden störungsfreien Betriebs Maßnahmen für die Wartung, beispielsweise Reinigungsmaßnahmen, Ersetzung der Sensorflüssigkeit von Verschleißteilen oder des Filters oder dergleichen, ermittelt und ausgegeben und/oder angezeigt und gegebenenfalls eingeleitet werden.

Es erweist sich des Weiteren als besonders vorteilhaft, wenn anhand der Informationen über die Dauer des verbleibenden störungsfreien Betriebs ein prädiktiver Zeitpunkt des Austauschs des Sensor ermittelt und vorzugsweise ausgegeben und/oder angezeigt wird. Diese Information des bevorzugten Zeitpunkts des Austauschs des Sensors kann natürlich auch in an sich beliebiger Weise an zentrale Rechen- und Steuereinheiten weitergegeben und dort in an sich beliebiger Weise verarbeitet werden.

Wenn vorstehend von Prüfparametem die Rede war, so waren hierunter an sich beliebige Zustandsdaten oder Zustandsinformationen des Sensors erfasst, welche sich im Zuge des Betriebs des Sensors verändern und in einem funktionalen Zusammenhang zur Standzeit des Sensors stehen.

Insbesondere bei potentiometrischen Sensoren, wie pH-Sensoren, O²- oder CO²-Sensoren erweist es sich als besonders vorteilhaft, wenn als Prüfparameter die Steilheit des Sensorsignals oder der Sensorsignale (Einzelsensor, Sensorkette oder Sensor-Array) in einem jeweiligen Prüfzustand des Sensors, also beispielsweise vorzugsweise während der zyklischen Kalibrierung des Sensors, erfasst und ausgewertet wird. Es wird hierunter also im Falle des beispielhaft genannten pH-Sensors die Steilheit der Messkettenspannung oder Zellen-EMK verstanden. Gleichermaßen könnte es sich um die hinterlegten Werte der Kalibrierfunktion eines photometrischen oder spektrometrischen Sensors handeln.

Des Weiteren kann es sich als vorteilhaft erweisen, wenn als Prüfparameter der Nullpunkt dieses Messkettensignals in einem jeweiligen Prüfzustand des Sensors erfasst und ausgewertet wird.

Eine besondere Bedeutung kommt auch der Erfassung und Auswertung des Innenwiderstands einer Elektrode als Prüfparameter zu, da dies in vorteilhafter Weise während des normalen Messbetriebs möglich ist, wohingegen die Ermittlung von Steilheit und Nullpunktslage nur bei unterbrochenem Messbetrieb , insbesondere während der zyklisch durchgeführten Kalibrierverfahren, möglich ist.

Erfindungsgemäss lässt sich die Veränderung des dynamischen Verhaltens vom Sensor selbst erzeugter Signale als Prüfparameter erfassen und auswerten. So kann beispielsweise die Anstiegs-/ beziehungsweise Abfallzeit bei der Signalerfassung oder die Signaleinschwingzeit oder das dynamische Verhalten des Rauschens des Sensors in der erfindungsgemäßen Weise zur Funktionsüberwachung verwendet werden.

Dynamisches Verhalten kann beispielsweise über ein Zyklovoltagramm ermittelt werden.

Des Weiteren erweist es sich als vorteilhaft, wenn abhängig vom Sensortyp wenigstens eines, vorteilhafterweise aber mehrere unterschiedliche Prüfparameter jeweils zyklisch erfasst werden. Die dann jeweils gewonnenen Informationen über die zu erwartende Entwicklung des Sensorverhaltens und über die Dauer des verbleibenden störungsfreien Betriebs können dann dahingehend verwertet werden, dass bei der Frage des Erreichens des Abnutzungsgrenzzeitpunkts diejenigen Prüfparameter Berücksichtigung finden, welche den nächstliegenden Abnutzungsgrenzzeitpunkt bedingen.

Es erweist sich auch als vorteilhaft, wenn sensorspezifische Grunddaten bei der Auswertung Verwendung finden. Anhand dieser sensorspezifischen Grunddaten, die aus einer Speichereinrichtung des Sensors, des Messwertumformers oder in sonstiger Weise, etwa über das Internet oder über Aktualisierungsdatenträger erhalten und verwendet werden, können beispielsweise bevorzugte und insbesondere ebenfalls hinterlegte Funktionen für den betreffenden Prüfparameter gewonnen und für die Durchführung des Verfahrens herangezogen werden.

In einer weiteren vorteilhaften Ausbildung des erfindungsgemäßen Verfahrens, können vorausschauende Hinweise zu Wartungs- oder Inspektionsmaßnahmen mit detaillierten Anweisungen, die Vorrichtung zu reinigen, auszuwechseln oder zu prüfen, gegeben werden, wenn sich herausstellt, dass ein für die Zukunft ermittelter Wert eines Prüfparameters in einem Warnbereich diesseits der Abnutzungsgrenze liegt. Ebenso können Empfehlungen für logistische Maßnahmen, welche die Lagerhaltung oder Materialbestellung betreffen, entsprechend hinterlegtem Erfahrungswissen ausgegeben oder in beliebiger Weise veranlasst werden.

Die Ergebnisse der Funktionsüberwachung könnten außerdem beispielsweise über prozesskompatible Schnittstellen, wie zum Beispiel Profibus, Foundation Fieldbus, Ethernet und dergleichen an eine Prozessleitstelle übermittelt werden.

Als Mittel zur gemeinsamen Nutzung der identischen Bedienoberfläche und zur Darstellung der ausgewerteten Prüfparameter, beispielsweise im Messumformer oder einem angeschlossenen PC, kann beispielsweise ein identischer Device Type Manager (DTM) verwendet werden.

Weitere Merkmale, Einzelheiten und Vorteile der Erfindung ergeben sich aus den Patentansprüchen, der zeichnerischen Darstellung und nachfolgenden Beschreibung einer bevorzugten Ausführungsform der Erfindung. In der Zeichnung zeigt:
- Figur 1: in schematischer Darstellung einen Teil einer Messeinrichtung;
- Figur 2: den Verlauf sensorspezifischer Prüfparameter über der Zeit.

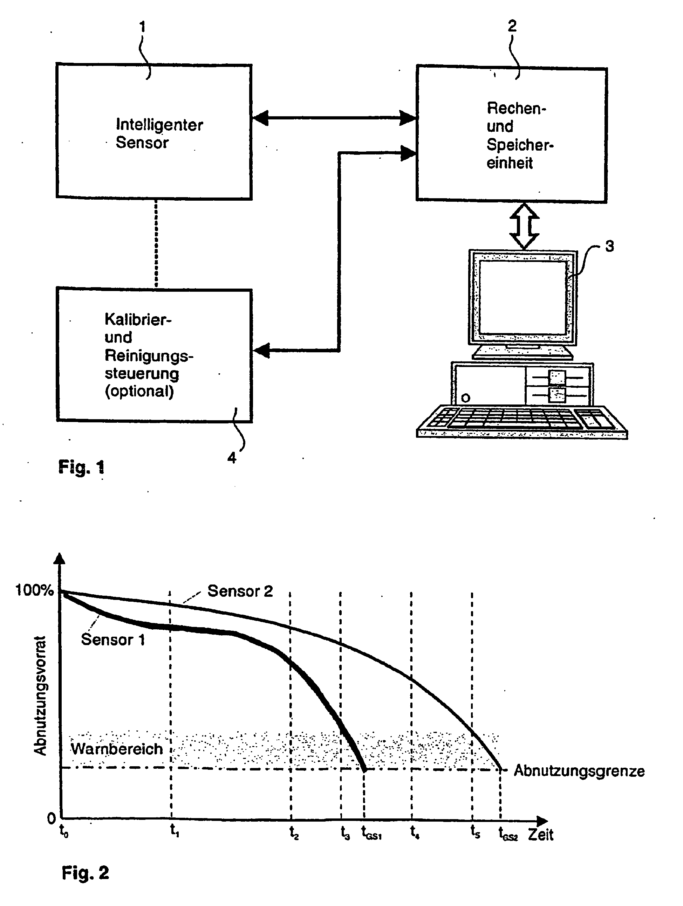

Figur 1 zeigt schematisch einen Teil einer Messeinrichtung, welche einen Sensor 1, eine Rechen- und Speichereinheit 2 sowie eine Anzeige- und Bedieneinheit 3 umfasst. Als optionale Komponente ist in das System eine automatisierbare Kalibrier- und Reinigungseinheit 4 mit zugehöriger Steuerung integriert.

Bei dem Sensor 1 kann es sich in vorteilhafter Weise um einen Sensor mit einer Kommunikationsschnittstelle und einem internen digitalen Speicher sowie zugehöriger prozessorgesteuerter Elektronik handeln; das erfindungsgemäße Verfahren ist aber auch unter Verwendung eines konventionellen Sensors mit analoger Signalausgabe an die Recheneinheit 2 eines Messwertumformers durchführbar.

Rechen- und Speichereinheit 2 sowie die Bedieneinheit 3 können in einem Feldgerät mit graphischem Display integriert sein, alternativ ist aber auch, wie dargestellt, ein PC verwendbar. Die Messeinrichtung besitzt auch eine Kommunikationsschnittstelle, um über die gängigen Kommunikationsverfahren via Internet oder lokalem Intranet mit einer zentralen Prozessleitstelle in Verbindung treten zu können oder von extern angesprochen werden zu können.

In Figur 2 ist der Verlauf sensorspezifischer Daten sensorspezifischer Prüfparameter über der Zeit aufgetragen. Beispielsweise durch äußere Einflüsse bedingt ist die Funktionsfähigkeit des Sensors zeitlich begrenzt; er unterliegt der Abnutzung. Sensorspezifisch gibt es verschiedene Einflussgrößen, wie die Belastung durch das Messmedium, die Belastung durch Temperatur und klimatische Einflüsse, aber auch die alterungsbedingte Abnutzung sowie sonstige chemische oder physikalische Vorgänge, die einen unterschiedlich schnellen Abbau des sog. Abnutzungsvorrats bis zur definierten Abnutzungsgrenze beeinflussen.

Für einen jeweiligen Prüfparameter ist in der Speichereinheit in Abhängigkeit vom spezifischen Sensortyp ein der Abnutzungsgrenze entsprechender Parameterwert hinterlegt. Ebenso ist daran angrenzend ein Warnbereich definiert und hinterlegt. Wie eingangs erwähnt, ist bei Erreichen der Abnutzungsgrenze die Funktionsfähigkeit der Messeinrichtung zwar noch gegeben, eine weitere Veränderung oder Verschlechterung der Sensorparameter hätte jedoch Fehlfunktionen zur Folge.

Für das Beispiel einer pH-Messkette ist in Figur 2 der Verlauf der sogenannten Elektrodensteilheit der Messkettenspannung dargestellt. Die Messeinrichtung erfasst dabei während eines jeweiligen Kalibriervorgangs die Messkettenspannungen und normiert die gewonnenen Werte in an sich bekannter Weise (zum Beispiel wie in DIN IEC 746-2 beschrieben). Die Ergebnisse werden dann in der Speichereinheit abgelegt.

Über ein sogenanntes Prädiktivprogramm zur Steilheitsüberwachung wird nun bei Vorliegen einer ersten Anzahl von Werten durch nichtlineare Interpolation eine Polynomfunktion ermittelt und ein erster prädiktiver Wert für die Abnutzungsgrenze in der Zukunft ermittelt. Dieser wird gespeichert. In Abhängigkeit von den Einsatzbedingungen und den daraus resultierenden Kalibrierzyklen, die sich im Tages- oder Wochenabstand abspielen können, werden die Daten ergänzt, d.h. es werden weitere Prüfparameter erfasst und deren Polynomfunktion ermittelt und jeweils durch Extrapolation im weitesten Sinne die Werte der Funktion zu weiteren Zeitpunkten tᵢ bestimmt. Sobald der betreffende Prüfparameter auf die definierte Größe des Warnbereichs absinkt, zum Beispiel bei Absinken des Elektrodensteilheitswerts auf 80% der theoretischen Steilheit der Messkettenspannung über der H⁺-Ionenkonzentration, wird der ebenfalls ermittelte Abnutzungsgrenzzeitpunkt als Warninformation ausgegeben. Wie in den Beispielen der beiden Sensoren der Figur 2 sind dies die Zeitpunkte (t_{GS1} und t_{GS2}).

Wie vorausgehend erwähnt, können alternativ oder zusätzlich als Prüf- oder Überwachungsparameter der Messkettenspannungsnullpunkt oder der Innenwiderstand der Glaselektrode und/oder der Bezugselektrode berücksichtigt werden.

Die erfindungsgemäße Maßnahme der Berücksichtigung der seitherigen Entwicklung von in zeitlichen Abständen erfassten Prüfparametern für die Ermittlung der zukünftig zu erwartenden Entwicklung des Sensorverhaltens, führt zu einer optimalen Ausnutzung der Funktionsfähigkeit des Sensors. Der Sensor braucht nicht schon lange vor Ablauf seiner Standzeit ausgewechselt zu werden, und andererseits wird das Risiko weitestgehend minimiert, dass der Sensor während des bestimmungsgemäßen Betriebs funktionsunfähig wird.

Gleichermaßen können vorausschauende Vorsorgemaßnahmen, z. B. Bereitstellung und Beschaffung von Ersatzmaterialien im Sinne einer optischen Instandhaltungsstrategie eingeleitet werden.

## Patentansprüche

1. Verfahren zur Funktionsüberwachung eines Sensors zur Messung und Überwachung von Zustandsparametern von Flüssigkeiten oder Gasen, im Bereich der Prozessmesstechnik, wobei der Sensor in zeitlichen Abständen in einen Prüfzustand versetzt wird und Prüfparameter erfasst werden oder Prüfparameter in zeitlichen Abständen im Zuge der Messwerterfassung erfasst werden, wobei die erfassten Prüfparameter gespeichert werden und wobei zur Durchführung der Funktionsüberwachung eine zeitliche Entwicklung der gespeicherten Prüfparameter ausgewertet wird und wobei daraus die zukünftige zu erwartende Entwicklung des Sensorverhaltens vorhergesagt wird und Informationen über die Dauer des verbleibenden störungsfreien Betriebs des Sensors gewonnen werden, **dadurch gekennzeichnet, dass** als Prüfparameter die Veränderung des dynamischen Verhaltens vom Sensor selbst erzeugter Signale erfasst und ausgewertet wird.

2. Verfahren nach Anspruch 1, wobei als Prüfparameter die Anstiegs- oder Abfallzeit bei der Signalerfassung, oder die Signaleinschwingzeit oder das dynamische Verhalten des Rauschens des Sensors zur Funktionsüberwachung verwendet wird.

3. Verfahren nach Anspruch 1 oder 2, **dadurch gekennzeichnet, dass** zum Auswerten der zeitlichen Entwicklung der gespeicherten Prüfparameter nichtlineare Interpolationsverfahren angewendet werden, um Funktionsparameter einer das Sensorverhalten beschreibenden Funktion zu gewinnen.

4. Verfahren nach einem der Ansprüche 1 bis 3, **dadurch gekennzeichnet, dass** für einen jeweiligen Sensor eine das erfahrungsgemäße Sensorverhalten wiedergebende Funktion vorgegeben und verwendet wird.

5. Verfahren nach Anspruch 4, **dadurch gekennzeichnet, dass** es sich bei der Funktion um eine Polynomfunktion handelt.

6. Verfahren nach einem oder mehreren der vorstehenden Ansprüche, **dadurch gekennzeichnet, dass** ein erster prädiktiver Wert für die Abnutzungsgrenze ermittelt wird.

7. Verfahren nach Anspruch 6, **dadurch gekennzeichnet, dass** geprüft wird, ob vor dem nächsten Erfassen von Prüfparametem die Abnutzungsgrenze erreicht wird.

8. Verfahren nach Anspruch 6 oder 7, **dadurch gekennzeichnet, dass** geprüft wird, ob ein prädiktiv gewonnener Wert des Prüfparameters innerhalb eines Warnbereichs diesseits der bisher definierten Abnutzungsgrenze liegt.

9. Verfahren nach einem oder mehreren der vorstehenden Ansprüche, **dadurch gekennzeichnet, dass** anhand der Informationen über die Dauer des verbleibenden störungsfreien Betriebs Maßnahmen für die Wartung ermittelt und ausgegeben und/oder angezeigt und gegebenenfalls eingeleitet werden.

10. Verfahren nach einem oder mehreren der vorstehenden Ansprüche, **dadurch gekennzeichnet, dass** anhand der Informationen über die Dauer des verbleibenden störungsfreien Betriebs ein prädiktiver Zeitpunkt des Austauschs des Sensors ermittelt und gegebenenfalls ausgegeben wird.

11. Verfahren nach einem oder mehreren der vorstehenden Ansprüche, **dadurch gekennzeichnet, dass** als Prüfparameter die Steilheit des Sensorsignals oder der Sensorsignale in einem jeweiligen Prüfzustand des Sensors erfasst und ausgewertet wird.

12. Verfahren nach einem oder mehreren der vorstehenden Ansprüche, **dadurch gekennzeichnet, dass** als Prüfparameter der Nullpunkt des Sensorsignals oder der Sensorsignale in einem jeweiligen Prüfzustand des Sensors erfasst und ausgewertet wird.

13. Verfahren nach einem oder mehreren der vorstehenden Ansprüche, **dadurch gekennzeichnet, dass** als Prüfparameter der Innenwiderstand einer Elektrode erfasst und ausgewertet wird.

14. Verfahren nach einem oder mehreren der vorstehenden Ansprüche, **dadurch gekennzeichnet, dass** mehrere verschiedene Prüfparameter erfasst und ausgewertet werden.

15. Verfahren nach einem oder mehreren der vorstehenden Ansprüche, **dadurch gekennzeichnet, dass** zur Auswertung sensorspezifische Grunddaten aus einer Speichereinrichtung des Sensors oder des Messwertumformers, über das Internet oder über Aktualisierungsdatenträger erhalten und verwendet werden.

## Claims

1. Procedure for the function monitoring of a sensor designed to measure and monitor condition parameters of liquids or gases in the field of process measuring technology, wherein the sensor is set to an inspection state at intervals and inspection parameters are recorded, or inspection parameters are recorded at intervals in the course of measured value logging, wherein the recorded inspection parameters are saved and wherein, to perform the function monitoring, a development of the saved inspection parameters over time is evaluated, and wherein this information is used to predict the future development in the sensor behavior that can be expected, and information is gathered about the duration of trouble-free sensor operation remaining, **characterized in that** the change in the dynamic behavior of signals generated by the sensor itself is recorded and evaluated as an inspection parameter.

2. Procedure as claimed in Claim 1, wherein the rise and fall time during signal recording, or the signal settling time, or the dynamic behavior of the noise of the sensor designed for function monitoring is used as an inspection parameter.

3. Procedure as claimed in Claim 1 or 2, **characterized in that** non-linear interpolation methods are employed to evaluate the development over time of the saved inspection parameters in order to obtain functional parameters of a function describing the sensor behavior.

4. Procedure as claimed in one of the Claims 1 to 3, **characterized in that** a function that reflects the empirical sensor behavior is prespecified and used for a particular sensor.

5. Procedure as claimed in Claim 4, **characterized in that** the function is a polynomial function.

6. Procedure as claimed in one or more of the previous claims, **characterized in that** a first predictive value is determined for the wear limit.

7. Procedure as claimed in Claim 6, **characterized in that** it is checked whether the wear limit will be reached before the next time the inspection parameters are recorded.

8. Procedure as claimed in Claim 6 or 7, **characterized in that** it is checked whether an inspection parameter value obtained predictively is within a warning range on this side of the wear limit defined thus far.

9. Procedure as claimed in one or more of the previous claims, **characterized in that** on the basis of the information about the duration of trouble-free operation remaining, maintenance measures are determined and output and/or displayed and initiated if necessary.

10. Procedure as claimed in one or more of the previous claims, **characterized in that** on the basis of the information about the duration of trouble-free operation remaining, a predictive time for the replacement of the sensor is determined and output where necessary.

11. Procedure as claimed in one or more of the previous claims, **characterized in that** the slope of the sensor signal or sensor signals in a particular sensor inspection state is recorded and evaluated as the inspection parameter.

12. Procedure as claimed in one or more of the previous claims, **characterized in that** the zero point of the sensor signal or sensor signals in a particular sensor inspection state is recorded and evaluated as the inspection parameter.

13. Procedure as claimed in one or more of the previous claims, **characterized in that** the internal resistance of an electrode is recorded and evaluated as the inspection parameter.

14. Procedure as claimed in one or more of the previous claims, **characterized in that** several different inspection parameters are recorded and evaluated.

15. Procedure as claimed in one or more of the previous claims, **characterized in that**, for the evaluation, sensor-specific basic data are obtained from a memory unit of the sensor or transducer, from the Internet or via update data carriers and are used.

## Revendications

1. Procédé destiné à la surveillance fonctionnelle d'un capteur destiné à la mesure et à la surveillance de paramètres d'état de liquides ou de gaz, dans le domaine de la technique de mesure de process, le capteur étant mis selon des intervalles de temps déterminés dans un état de contrôle et les paramètres de contrôle étant mesurés ou les paramètres de contrôle étant mesurés selon des intervalles de temps déterminés dans le cadre de l'acquisition des valeurs mesurées, les paramètres de contrôle mesurés étant enregistrés et une évolution dans le temps des paramètres de contrôle enregistrés étant évaluée en vue de l'exécution de la surveillance fonctionnelle, et l'évolution future escomptée du comportement du capteur étant pronostiquée sur la base de cette évaluation, et des informations sur la durée du fonctionnement sans dérangement restant du capteur étant obtenues, **caractérisé en ce qu'**en tant que paramètre de contrôle, le changement du comportement dynamique de signaux générés par le capteur lui-même est mesuré et évalué.

2. Procédé selon la revendication 1, pour lequel est utilisé en tant que paramètre de contrôle le temps de montée ou de descente pour la mesure des signaux, ou le temps de réponse des signaux ou le comportement dynamique du bruit du capteur destiné à la surveillance fonctionnelle.

3. Procédé selon la revendication 1 ou 2, **caractérisé en ce que** sont appliqués, pour l'évaluation de l'évolution dans le temps des paramètres de contrôle enregistrés, des procédés d'interpolation non linéaires afin d'obtenir des paramètres fonctionnels d'une fonction décrivant le comportement du capteur.

4. Procédé selon la revendication 1 à 3, **caractérisé en ce que**, pour un capteur respectif, une fonction reflétant le comportement empirique du capteur est prédéfini et utilisé.

5. Procédé selon la revendication 4, **caractérisé en ce que**, concernant la fonction, il s'agit d'une fonction polynomiale.

6. Procédé selon l'une des revendications précédentes, **caractérisé en ce qu'**une première valeur prédictive est déterminée pour la limite d'usure.

7. Procédé selon la revendication 6, **caractérisé en ce qu'**est contrôlé le fait que la limite d'usure est atteinte avant la prochaine acquisition des paramètres de contrôle.

8. Procédé selon la revendication 6 ou 7, **caractérisé en ce qu'**est contrôlé le fait qu'une valeur prédictive obtenue du paramètre de contrôle se situe dans une plage d'avertissement en deçà de la limite d'usure jusqu'ici définie.

9. Procédé selon l'une des revendications précédentes, **caractérisé en ce qu'**au moyen des informations concernant la durée du fonctionnement sans dérangement restant sont déterminées et émises et/ou affichées, et le cas échéant, prises des mesures pour la maintenance.

10. Procédé selon l'une des revendications précédentes, **caractérisé en ce qu'**au moyen des informations concernant la durée du fonctionnement sans dérangement restant est déterminé et, le cas échéant, est émis un instant prédictif du remplacement du capteur.

11. Procédé selon l'une des revendications précédentes, **caractérisé en ce qu'**en tant que paramètre de contrôle est déterminée et évaluée la pente du signal de capteur ou des signaux de capteur dans un état de contrôle respectif du capteur.

12. Procédé selon l'une des revendications précédentes, **caractérisé en ce qu'**en tant que paramètre de contrôle est déterminé et évalué le zéro du signal de capteur ou des signaux de capteur dans un état de contrôle respectif du capteur.

13. Procédé selon l'une des revendications précédentes, **caractérisé en ce qu'**en tant que paramètre de contrôle est déterminée et évaluée la résistance interne d'une électrode.

14. Procédé selon l'une des revendications précédentes, **caractérisé en ce que** plusieurs paramètres de contrôle différents sont mesurés et évalués.

15. Procédé selon l'une des revendications précédentes, **caractérisé en ce que**, pour l'évaluation, des données de base sont obtenues à partir d'un dispositif de stockage du capteur ou du transducteur de mesure, par le biais d'Internet ou du support de données d'actualisation, et sont utilisées.
